# EUROPEAN PATENT APPLICATION

(11) **EP 3 456 812 A1**
(43) Date of publication of application: **20.03.2019**
(21) Application number: 17815066.0
(22) Date of filing: 18.05.2017
(51) Int. Cl.: C12M 1/34, G01N 33/48, G06Q 50/22, G06T 1/00

(54) **INFORMATION PROCESSING DEVICE, INFORMATION PROCESSING METHOD, AND PROGRAM**

(30) Priority: 21.06.2016 JP 2016122494
(71) Applicant: Sony Corporation, Tokyo 108-0075 (JP)
(72) Inventor: WU, Jianing, Tokyo 108-0075 (JP); SHINODA, Masataka, Tokyo 108-0075 (JP); AKAMA, Taketo, Tokyo 108-0075 (JP)
(74) Representative: Witte, Weller & Partner Patentanwälte mbB
(86) International application number: PCT/JP2017/018683
(87) International publication number: WO 2017/221597

(57) **Abstract**

An information processing apparatus according to an embodiment of the present technology includes a first extraction unit, a second extraction unit, and a comparison unit. The first extraction unit extracts, from observation data of a cell before transfer, a first feature quantity of the cell before transfer. The second extraction unit extracts, from observation data of a cell after transfer, a second feature quantity of the cell after transfer. The comparison unit compares the extracted first feature quantity and the extracted second feature quantity with each other.

## Description

### Technical Field

The present technology relates to an information processing apparatus, an information processing method, and a program that can be used for transferring a fertilized egg and the like.

### Background Art

As described in Patent Literature 1, in the fields of fertility treatments, assisted reproductive technology, and the like, there is a problem in that specimens of sperm, fertilized eggs, or the like are mixed up. In a management system described in Patent Literature 1, an identification tag on which a dotted pattern readable by an electronic pen is printed is attached to a vessel that contains a specimen. In a case where transfer work is performed, necessary information is described on a vessel of a transfer source and a vessel of a transfer destination with the electronic pen. In this case, the information read from the identification tags is sent to a terminal apparatus. Then, the terminal apparatus determines whether or not transfer is correctly performed. With this configuration, worker's visual check through the description with the electronic pen and automatic check by the management system are both performed, and highly reliable management can be achieved (paragraphs [0002], [0022], [0023], and [0037] to [0045] of specification, Fig. 1, and the like of Patent Literature 1).

### Citation List

### Patent Literature

Patent Literature 1: Japanese Patent Application Laid-open No. 2014-67216

### Disclosure of Invention

### Technical Problem

It is extremely important to prevent mix-up of cells such as fertilized eggs, and it is desirable to provide a useful technology with which a desired cell can be transferred to a correct position.

In view of the above-mentioned circumstances, it is an object of the present technology to provide an information processing apparatus, an information processing method, and a program with which mix-up and the like of cells can be sufficiently prevented. Solution to Problem

In order to accomplish the above-mentioned object, an information processing apparatus according to an embodiment of the present technology includes a first extraction unit, a second extraction unit, and a comparison unit.

The first extraction unit extracts, from observation data of a cell before transfer, a first feature quantity of the cell before transfer.

The second extraction unit extracts, from observation data of a cell after transfer, a second feature quantity of the cell after transfer.

The comparison unit compares the extracted first feature quantity and the extracted second feature quantity with each other.

In this information processing apparatus, the first feature quantity extracted from the observation data of the cell before transfer and the second feature quantity extracted from the observation data of the cell after transfer are compared with each other. With this configuration, it is possible to easily determine whether the cell before transfer and the cell after transfer are the same cells. As a result, it is possible to sufficiently prevent mix-up and the like of cells.

The observation data of the cell may be an image of the cell, which is taken by one or more imaging apparatuses.

By extracting the first feature quantity and the second feature quantity from the images of the cells and comparing the first feature quantity and the second feature quantity with each other, it is possible to sufficiently prevent mix-up and the like of cells.

The one or more imaging apparatuses may include at least one of a visible-light camera, an infrared camera, or a polarization camera.

It is possible to extract, on a basis of images taken by those cameras, a feature quantity with which the cell can be identified.

The observation data of the cell may be a plurality of images obtained by imaging the cell in a plurality of directions.

With this configuration, it is possible to extract a feature quantity capable of sufficiently identifying the cell.

The observation data of the cell may be a moving image obtained by imaging the cell in a time series.

With this configuration, it is possible to extract a feature quantity capable of sufficiently identifying the cell.

The first extraction unit and the second extraction unit may respectively extract the first feature quantity and the second feature quantity on a basis of a predetermined machine learning algorithm.

With this configuration, it is possible to extract a feature quantity capable of sufficiently identifying the cell.

The first extraction unit may detect, from the observation data of the cell before transfer, a posture of the cell before transfer and extract the first feature quantity on a basis of the detected posture. In this case, the second extraction unit may detect, from the observation data of the cell after transfer, a posture of the cell after transfer and extract the second feature quantity on a basis of the detected posture.

With this configuration, it is possible to improve the accuracy of the comparison result of the comparison unit.

The information processing apparatus may further include a determination unit that determines whether or not the transfer of the cell is normal on a basis of a comparison result of the comparison unit.

With this configuration, it is possible to sufficiently prevent mix-up and the like of cells.

The first extraction unit may extract a feature quantity of a vessel before transfer, the feature quantity relating to the vessel in which the cell before transfer is put. In this case, the second extraction unit extracts a feature quantity of a vessel after transfer, the feature quantity relating to the vessel in which the cell after transfer is put. Further, the determination unit may determine whether or not the transfer of the cell is normal on a basis of the extracted feature quantity of the vessel before transfer and the extracted feature quantity of the vessel after transfer.

With this configuration, it is possible to sufficiently prevent mix-up and the like of cells.

The information processing apparatus may further include a third extraction unit that extracts, from observation data of a cell being transferred, a third feature quantity of the cell being transferred.

With this configuration, it is possible to sufficiently prevent mix-up and the like of cells.

The information processing apparatus may further include a notification unit that makes notification of a determination result of the determination unit.

With this configuration, it is possible to sufficiently prevent mix-up and the like of cells.

The notification unit may make notification of the determination result by using at least one of an image, a sound, or a vibration.

With this configuration, it is possible to sufficiently makes notification of the determination result.

The notification unit may output a notification image including the determination result.

With this configuration, it is possible to sufficiently makes notification of the determination result.

The notification image may include at least one of a transfer condition of the cell or a guide image for guiding for a transfer operation of the cell.

The transfer condition of the cell or the guide image is displayed, and thus it is possible to sufficiently prevent mix-up and the like of cells.

The notification unit may display a correction image for correcting to normal transfer if it is determined that the transfer of the cell is not normal.

With this configuration, it is possible to guide to a transfer operation of a correct cell.

An information processing method according to an embodiment of the present technology is an information processing method to be executed by a computer system and includes extracting, from observation data of a cell before transfer, a first feature quantity of the cell before transfer.

A second feature quantity of a cell after transfer is extracted from observation data of the cell after transfer.

The extracted first feature quantity and the extracted second feature quantity are compared with each other.

A program according to an embodiment of the present technology causes a computer system to execute the following steps.

A step of extracting, from observation data of a cell before transfer, a first feature quantity of the cell before transfer.

A step of extracting, from observation data of a cell after transfer, a second feature quantity of the cell after transfer.

A step of comparing the extracted first feature quantity and the extracted second feature quantity with each other.

### Advantageous Effects of Invention

As described above, in accordance with the present technology, it is possible to sufficiently prevent mix-up and the like of cells. It should be noted that the effects described here are not necessarily limitative and may be any effect described in the present disclosure.

### Brief Description of Drawings

[Fig. 1] A schematic diagram showing a configuration example of a culture system according to a first embodiment.
[Fig. 2] A diagram showing a flow of a normality/abnormality determination example of a transfer operation according to the present technology.
[Fig. 3] A schematic diagram showing examples of a notification image.
[Fig. 4] A schematic diagram showing examples of the notification image.
[Fig. 5] A schematic diagram showing examples of the notification image.
[Fig. 6] A schematic diagram showing a culture vessel and an imaging unit according to a second embodiment.
[Fig. 7] A diagram for describing a posture of a fertilized egg.
[Fig. 8] A schematic diagram showing a transfer tool to be used in a culture system according to a third embodiment.
[Fig. 9] A perspective view showing a configuration example of a head-mounted display (HMD) as an information processing apparatus according to another embodiment.

### Mode(s) for Carrying Out the Invention

Hereinafter, embodiments according to the present technology will be described with reference to the drawings.

### <First Embodiment>

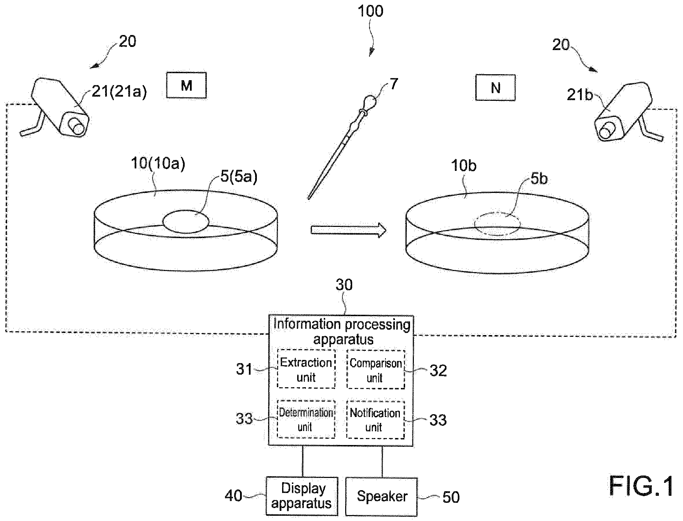

Fig. 1 is a schematic diagram showing a configuration example of a culture system according to a first embodiment of the present technology. In this embodiment, a culture system 100 cultures a fertilized egg of an organism in the field of animal husbandry or the like. The present technology is not limited thereto, and the present technology is applicable to any other cells.

Examples of the cell to be cultured can include an unfertilized egg cell (egg), an embryo, and the like of an organism in the field of animal husbandry or the like, biological specimens extracted from living bodies such as stem cells, immune cells, and cancer cells in the field of regenerative medicine, pathobiology, or the like, and the like. Herein, the term "cell" at least conceptionally includes a single cell and an aggregate of a plurality of cells.

As shown in Fig. 1, the culture system 100 includes a plurality of culture vessels 10, an imaging unit 20, an information processing apparatus 30, a display apparatus 40, and a speaker 50.

The culture vessel 10 is configured to be capable of containing a culture medium and a fertilized egg 5, and has light transmittance such that the fertilized egg 5 can be imaged from the outside. The shape of the culture vessel 10 is not particularly limited. For example, a flat-dish shape such as a Petri dish is used.

A culture vessel 10a of the plurality of culture vessels 10 is arranged at a point M. Further, a culture vessel 10b is arranged at a point N different from the point N. An embryologist (user) transfers the fertilized egg 5 from the culture vessel 10a to the culture vessel 10b by using a transfer tool 7 such as a pipette.

The transfer operation of the fertilized egg 5 is performed in a case of changing a component of the culture medium or in a case of changing the culture vessel 10 in the culture process of the fertilized egg 5, for example. Further, the fertilized egg 5 is also transferred to a dedicated vessel or the like in a case of visually checking the fertilized egg 5, in a case of transferring the fertilized egg 5 to a microscope or the like, or in a case of filling a straw tube for freezing with the fertilized egg 5, for example.

The imaging unit 20 includes an imaging apparatus 21 arranged at the point M and an imaging apparatus 21b arranged at the point N. The imaging apparatus 21a images the fertilized egg 5 before transfer, which is put in the culture vessel 10a, and the culture vessel 10a and generates an image of the fertilized egg 5 and the culture vessel 10a. The imaging apparatus 21b images the fertilized egg 5 after transfer, which is put in the culture vessel 10b, and the culture vessel 10b and generates an image of the fertilized egg 5 and the culture vessel 10b.

Hereinafter, the images taken by the imaging apparatuses 21a and 21b will be sometimes referred to as an image before transfer and an image after transfer. Further, the fertilized egg 5 before transfer and the fertilized egg 5 after transfer will be sometimes referred to as a fertilized egg 5a and a fertilized egg 5b.

The image taken by each imaging apparatus 21 includes both of a plurality of still images taken at predetermined imaging intervals and a moving image taken in a time series. Irrespective of whether the image of the fertilized egg 5 and the culture vessel 10 is the still image or the moving image, the present technology is applicable.

It should be noted that although the one fertilized egg 5 and the one culture vessel 10 are shown at each of the points M and N in Fig. 1, a plurality of culture vessels 10 are often arranged at each point and transfer operations are performed on a plurality of fertilized eggs 5. In this case, for the plurality of fertilized eggs 5a at the point M, a plurality of culture vessels 10b of transfer destinations are arranged at the point N. Then, the imaging apparatuses 21a and 21b respectively take a plurality of images before transfer and a plurality of images after transfer.

In this embodiment, the image of the fertilized egg 5a before transfer corresponds to observation data of the fertilized egg 5a before transfer. Further, the image of the fertilized egg 5b after transfer corresponds to observation data of the fertilized egg 5b after transfer. It should be noted that a "cell before transfer" is a cell that the embryologist wishes to transfer, and in this embodiment, the fertilized egg 5a put in the culture vessel 10a corresponds thereto. On the other hand, a "cell after transfer" is a cell which has been transferred by the embryologist's transfer operation, and in this embodiment, the fertilized egg 5b put in the culture vessel 10b corresponds thereto.

For example, in a case of transferring the plurality of fertilized eggs 5a, there is a possibility that the embryologist may mix up and transfer the fertilized eggs 5a. That is, there is a possibility that the embryologist may transfer a fertilized egg 5a different from the fertilized egg that the embryologist wishes to transfer, for example, another fertilized egg 5a put in another culture vessel 10a at the point M, to the culture vessel 10b in error. In this case, the "cell before transfer" and the "cell after transfer" are different from each other. That is, in the present disclosure, the "cell before transfer" and the "cell after transfer" are not always the same cells. As will be described later, by carrying out the present technology, it is possible to sufficiently prevent mix-up and the like in which these would be different.

For example, visible-light cameras including image sensors such as complementary metal-oxide semiconductor (CMOS) sensors and charge coupled device (CCD) sensors are used as the imaging apparatuses 21a and 21b.

The display apparatus 40 is a display device using a liquid-crystal, electro-luminescence (EL), or the like, for example. The display apparatus 40 displays various images output from the information processing apparatus 30. The speaker 50 outputs a sound on a basis of audio data output from the information processing apparatus 30.

The information processing apparatus 30 controls operations of the respective blocks of the culture system 100. For example, as shown in Fig. 1, the imaging apparatuses 21a and 21b, the display apparatus 40, and the speaker 50 are connected to the information processing apparatus 30 via a wire or wirelessly. Then, the imaging operations of the imaging apparatuses 21a and 21b, the display operation of the display apparatus 40, and the sound output operation of the speaker 50 are respectively controlled.

The information processing apparatus 30 includes hardware necessary for a computer configuration, such as a central processing unit (CPU), a read only memory (ROM), a random access memory (RAM), and a hard disk drive (HDD). Although a personal computer (PC) is, for example, used as the information processing apparatus 30, any other computers may be used.

The CPU loads a program according to the present technology, which is stored in the ROM or HDD, to the RAM and executes it. In this manner, an extraction unit 31, a comparison unit 32, a determination unit 33, and a notification unit 34 that are functional blocks shown in Fig. 1 are realized. Then, an information processing method according to the present technology is executed by these functional blocks. It should be noted that dedicated hardware may be used as appropriate in order to realize each of the functional blocks.

The program is installed to the information processing apparatus 30 via any recording medium, for example. Alternatively, the program may be installed via the Internet or the like.

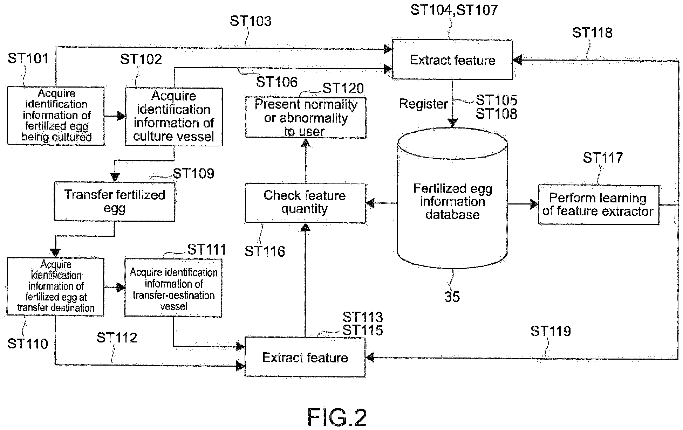

Fig. 2 is a diagram showing a flow of a normality/abnormality determination example of the transfer operation according to the present technology. First, the imaging apparatus 21a at the point M takes an image before transfer, which includes the fertilized egg 5a and the culture vessel 10a, and outputs the image to the information processing apparatus 30.

The extraction unit 31 acquires an image of the fertilized egg 5a as identification information on a basis of the image before transfer (Step 101). Further, on a basis of the image before transfer, an image of the culture vessel 10a is acquired as the identification information (Step 102). For example, pre-processing including image normalization, position adjustment of the fertilized egg 5a, filtering for emphasizing the shape, and the like is performed on the image before transfer output from the imaging apparatus 21a. The pre-processing result is acquired as the images of the fertilized egg 5a and the culture vessel 10a. It should be noted that the image before transfer may be acquired as the identification information as it is.

The extraction unit 31 extracts a first feature quantity of the fertilized egg 5a before transfer on a basis of the image of the fertilized egg 5a (Steps 103 and 104). The first feature quantity is extracted on a basis of, for example, various states of the fertilized egg 5a such as the size, the shape, the sphericity, and the cleavage degree (rate) of the fertilized egg 5a, the form of each blastomere, the balance thereof, and the amount of fragmentation. Further, in a case where the moving image is taken as the image of the fertilized egg 5a, a change and the like of the fertilized egg 5a in a time series can also be reflected to extraction of the first feature quantity.

Specific feature extraction processing for extracting the first feature quantity is not limited, and any extraction processing may be used. In this embodiment, a machine learning algorithm is used, and this point will be described later.

The extracted first feature quantity, an ID of the fertilized egg 5a, and the image of the fertilized egg 5a, which is used for the feature extraction, are registered in a fertilized egg information database (DB) 35 (Step 105). It should be noted that the ID of the fertilized egg 5a is set in advance for conducting the culture. Further, the fertilized egg information DB 35 is constructed by a storage unit such as the HDD of the information processing apparatus 30. As a matter of course, the fertilized egg information DB 35 may be constructed by another storage apparatus and may be connected to the information processing apparatus 30 via a network or the like

The extraction unit 31 extracts a feature quantity of the culture vessel 10a before transfer on a basis of the image of the culture vessel 10a (Steps 106 and 107). The feature quantity of the culture vessel 10a is extracted on a basis of, for example, the shape, a flaw, or the like of the culture vessel 10a. Alternatively, in a case where the culture vessel 10a is provided with identification information such as a label capable of identifying it, that identification information may be extracted as the feature quantity of the culture vessel 10a. The extracted feature quantity and the image of the culture vessel 10a are stored in the fertilized egg information DB 35 in association with the first feature quantity or the like of the fertilized egg 5a (Step 108).

The first feature quantity and the feature quantity of the culture vessel 10a are extracted in such a manner that the extraction unit 31 functions as a first extraction unit.

The embryologist performs a transfer operation by using the transfer tool 7. In this manner, the fertilized egg 5a is transferred to the culture vessel 10b at the point N that is a transfer destination (Step 109).

The imaging apparatus 21b at the point N takes an image after transfer of the fertilized egg 5b and the culture vessel 10b after transfer, and outputs the image to the information processing apparatus 30. The extraction unit 31 acquires an image of the fertilized egg 5b as the identification information on a basis of the image after transfer (Step 110). Further, on a basis of the image after transfer, an image of the culture vessel 10b is acquired as the identification information (Step 111).

The extraction unit 31 extracts a second feature quantity of the fertilized egg 5b after transfer on a basis of the image of the fertilized egg 5b (Steps 112 and 113). Further, the extraction unit 31 extracts a feature quantity of the culture vessel 10b after transfer on a basis of the image of the culture vessel 10b (Steps 114 and 115). The second feature quantity and the feature quantity of the culture vessel 10b are extracted in such a manner that the extraction unit 31 functions as a second extraction unit.

The extracted second feature quantity and the extracted feature quantity of the culture vessel 10b are checked against the first feature quantity and the feature quantity of the culture vessel 10a which are stored in the fertilized egg information DB 35 (Step 116). That is, the comparison unit 32 compares the first feature quantity and the second feature quantity with each other. On a basis of that comparison result, the determination unit 33 determines whether or not the transfer of the fertilized egg 5 is normal. Further, the determination unit 33 determines the normality or abnormality of the transfer operation on a basis of the feature quantity of the culture vessel 10a and the feature quantity of the culture vessel 10b.

For example, the comparison unit 32 detects a difference between the first feature quantity and the second feature quantity. If the difference between the feature quantities is smaller than a predetermined threshold, the determination unit 33 determines that the fertilized egg 5a before transfer and the fertilized egg 5b after transfer are the same fertilized eggs. That is, it is determined that mix-up of the fertilized eggs 5 has not occurred.

If the difference between the feature quantities is equal to or larger than the threshold, it is determined that the fertilized egg 5a before transfer and the fertilized egg 5b after transfer are different fertilized eggs and mix-up has occurred. A specific algorithm for the matching determination of the fertilized eggs 5 using the first feature quantity and the second feature quantity is not limited and may be set as appropriate. For example, a determination algorithm depending on the extraction algorithm for the first feature quantity and the second feature quantity may be set.

Before the transfer operation of the fertilized egg 5a, the image of the culture vessel 10b of the transfer destination at the point N may be taken and the feature quantity of the culture vessel 10b may be extracted. That is, the feature quantity of the culture vessel 10b to which the fertilized egg 5a should be transferred may be extracted in advance. With this configuration, it is possible to determine whether or not the fertilized egg 5a is transferred to the correct culture vessel 10b of the transfer destination.

That is, if the extracted feature quantity of the culture vessel 10b is approximately equal to the feature quantity of the culture vessel 10b to which the fertilized egg 5a should be transferred (e.g., a difference is equal to or smaller than a threshold), it is determined that the fertilized egg 5a is transferred to the correct culture vessel 10b. If the feature quantity of the culture vessel 10b is unequal to the feature quantity of the culture vessel 10b to which the fertilized egg 5a should be transferred, it is determined that the fertilized egg 5a is transferred to another culture vessel 10b at the point N in error.

Further, the feature quantity of the culture vessel 10a containing the fertilized egg 5a that should be transferred may be stored in advance. For example, it is assumed that the fertilized egg ID and the feature quantity of the culture vessel 10a are stored in association with each other. In this case, for example, the extracted feature quantity of the culture vessel 10a is checked against the stored feature quantity. If the feature quantities are approximately equal, it can be determined that it is transfer from the correct culture vessel 10a. If the feature quantities are unequal, it can be determined that it is transfer of the incorrect fertilized egg 5a from the other culture vessel 10a.

As shown in Fig. 2, in this embodiment, a feature extractor, i.e., the extraction unit 31 is updated on a basis of a predetermined machine learning algorithm. A parameter (coefficient) for feature quantity extraction is calculated by machine learning on a basis of the first feature quantity, the ID of the fertilized egg 5a, and the image of the fertilized egg 5a which are stored in the fertilized egg information DB 35 (Step 117). The calculated parameter is output to the extraction unit 31 and is used for extraction of the first feature quantity and the second feature quantity (Steps 118 and 119).

With this configuration, the extraction unit 31 is improved and the first feature quantity and the second feature quantity having higher identification performance can be extracted. As a result, even if many fertilized eggs 5 are treated, it is possible to sufficiently identify each fertilized egg 5, and it is possible to improve the accuracy of determination as to the normality or abnormality of the transfer operation.

The machine learning algorithm to be used is not limited. For example, a machine learning algorithm using a neural network such as a recurrent neural network (RNN), a convolutional neural network (CNN), and multilayer perceptron (MLP) is used. In addition, any machine learning algorithm executing supervised learning, unsupervised learning, semisupervised learning, reinforcement learning, or the like may be used.

The notification unit 34 makes notification of the determination result of the determination unit 33 (Step 120). In this embodiment, notification of the determination result is made by using an image and a sound. Specifically, the notification unit 34 generates an notification image including the determination result and the notification image is displayed on the display apparatus 40. Further, a sound depending on the determination result is mainly output from the speaker 50.

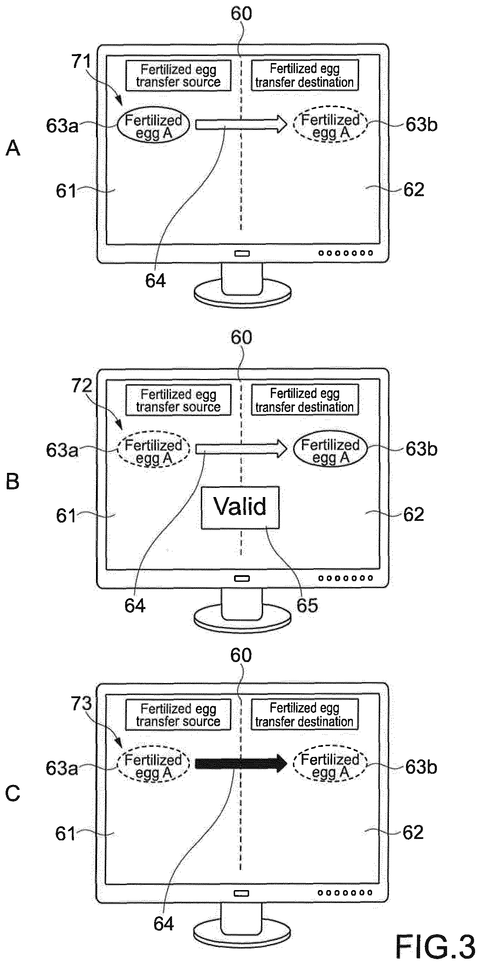

Figs. 3 to 5 are schematic diagrams showing examples of the notification image. In the following description of a notification image 60, the fertilized egg 5a before transfer and the fertilized egg 5b after transfer will be simply referred to as a fertilized egg A before transfer and a fertilized egg A after transfer.

The notification image 60 includes a transfer source region 61 indicating a transfer source (point M) of the fertilized egg 5 and a transfer destination region 62 indicating a transfer destination (point N). An image 63a of the fertilized egg A before transfer is displayed in the transfer source region 61 and an image 63b of the fertilized egg A after transfer is displayed in the transfer source region.

In the notification image 60 shown in A of Fig. 3, a first transfer state image 71 indicating a state before the transfer operation is performed is displayed. It can be said that the first transfer state image 71 is an image indicating a transfer operation scheduled to be performed. In the first transfer state image 71, the images 63a and 63b of the fertilized eggs A before transfer and after transfer and an arrow 64 extending from the image 63a of the fertilized egg A to the image 63b of the fertilized egg A are displayed.

The image 63a of the fertilized egg A before transfer is shown such that the presence of the fertilized egg A at the transfer source can be recognized (in this example, a solid-line frame is shown). On the other hand, the image 63b of the fertilized egg A after transfer is shown such that the absence of the fertilized egg A at the transfer destination can be recognized (in this example, a broken-line frame is shown).

By checking the first transfer state image 71, the embryologist can check the transfer operation to be performed. Alternatively, the embryologist can also check a transfer operation to be performed by another person. Further, the embryologist can also check that the fertilized egg A has not yet been transferred.

It should be noted that the first transfer state image is generated on a basis of an input to the information processing apparatus 30, which is made by embryologist who performs the transfer operation, for example. Alternatively, the culture vessels 10a and 10b may be placed at predetermined positions of the points M and N, and the first transfer state image 71 may be thus automatically displayed on a basis of images taken by the imaging apparatuses 21a and 21b. Alternatively, the first transfer state image 71 may be automatically displayed on a basis of other information regarding a culture step.

As shown in B of Fig. 3, if the determination unit 33 determines that the normal transfer operation is performed, a second transfer state image 72 indicating a state in which the fertilized egg A is transferred from the transfer source region 61 to the transfer destination region 62 is displayed. That is, the image 63a of the fertilized egg A before transfer is displayed with a broken-line frame and the image 63b of the fertilized egg A after transfer is displayed with a solid-line frame.

Further, as a determination result image 65 indicating that the transfer is normally performed, a text image "Valid" is displayed. Further, a sound indicating that the transfer is normally performed is output from the speaker 50. With this configuration, the embryologist can easily check that the transfer operation is normally performed.

It should be noted that if pick-up of the fertilized egg A can be detected on a basis of the image of the imaging apparatus 21a at the transfer source, a third transfer state image 73 in which the fertilized egg A is being transferred, which is shown in C of Fig. 3, may be displayed. The images 63a and 63b of the fertilized eggs A before transfer and after transfer are both displayed with broken-line frames and the arrow 64 is colored. With this configuration, it is possible to easily grasp that the fertilized egg A is being transferred. It should be noted that a sound indicating that the fertilized egg A is being transferred may be output from the speaker 50.

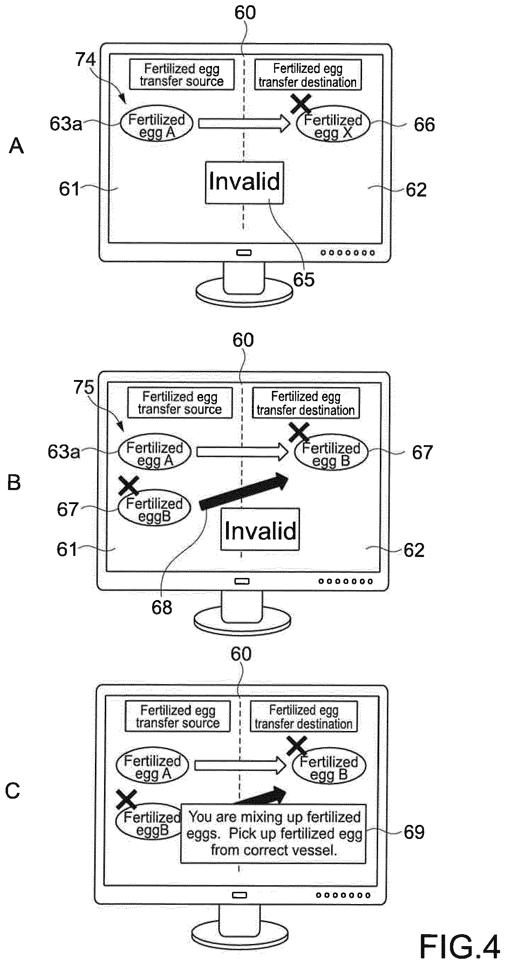

As shown in A of Fig. 4, if the determination unit 33 determines that the fertilized eggs before transfer and after transfer are different fertilized eggs, a fourth transfer state image 74 indicating a state in which mix-up occurs is displayed. For example, the image 63a of the fertilized egg A at the transfer source is displayed with a solid-line frame. In the transfer destination region 62, an image 66 of an unknown fertilized egg X, which is not the fertilized egg A, is displayed such that the fact that it is an incorrect fertilized egg can be recognized (in this example, an x mark is added).

Further, as a determination result image 65 indicating that the transfer is not normally performed, a text image "Invalid" is displayed. Moreover, an alarm sound or the like indicating that the transfer is not normally performed is output from the speaker 50. With this configuration, the embryologist can easily check that mix-up occurs and can quickly correct the transfer operation.

If in the checking step at Step 116 of Fig. 2, it is determined that the fertilized eggs before transfer and after transfer do not match each other, a fertilized egg having a feature quantity approximately equal to the feature quantity of the fertilized egg X transferred in error may be detected from the fertilized egg information DB 35. As shown in B of Fig. 4, if the fertilized egg X transferred in error is detected, a fifth transfer state image 75 including an image 67 of that fertilized egg (in this example, a fertilized egg B) is displayed.

For example, in the transfer source region 61, the image 67 of the fertilized egg B is displayed such that it is an incorrect fertilized egg. An arrow 68 is displayed such that the fact that an incorrect transfer operation is performed can be checked from the image of the fertilized egg B (e.g., it is shown in predetermined color). In the transfer destination region 62, the image 67 of the fertilized egg B is displayed together with an x mark at a position that is the transfer destination of the fertilized egg A. With this configuration, it is possible to easily grasp that the fertilized egg B is transferred to the culture vessel 10b to which the fertilized egg A should be transferred.

Further, a sound depending on the fifth transfer state image 75 may be output from the speaker 50. The same applies to a case of displaying other transfer state images shown below.

The embryologist can quickly restore the original state by returning the fertilized egg B, on which the transfer operation is performed, to the culture vessel 10a of the transfer source. Alternatively, the embryologist can also transfer the fertilized egg B to the culture vessel 10b to which the fertilized egg B at the transfer destination should be transferred. The embryologist can quickly and accurately correct the transfer operation in this manner. Thus, it is possible to sufficiently prevent mix-up and the like of the fertilized eggs.

It should be noted that as shown in C of Fig. 4, for example, a correction image 69 for correcting to normal transfer, which includes an indication for urging to correct the operation, may be displayed. In the example shown in C of Fig. 4, the text image is displayed, though not limited thereto. An image using an arrow or the like may be displayed as the correction image 69.

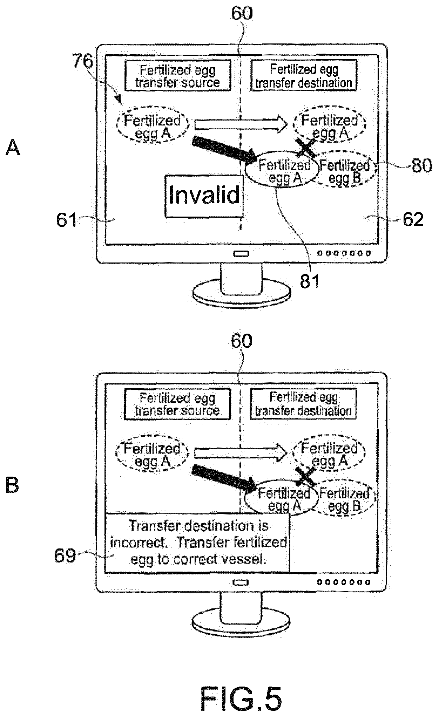

As shown in A of Fig. 5, if it is determined that the fertilized egg A is not transferred to the correct culture vessel 10b of the transfer destination, a sixth transfer state image 76 is displayed. In the example shown in A of Fig. 5, an indication that the culture vessel 10b of the transfer destination is the culture vessel 10b to which the fertilized egg B should be transferred is displayed such that it can be checked. That is, an image 81 of the fertilized egg A with a solid-line frame is displayed so as to overlap an image 80 of the fertilized egg B with a broken-line frame. With this configuration, the embryologist can return the fertilized egg A to the original culture vessel 10a or easily perform correction such as transferring the fertilized egg A to the correct culture vessel 10b. It should be noted that as shown in B of Fig. 5, a correction image 69 for correcting to normal transfer may be displayed.

In this embodiment, the above-mentioned first to sixth transfer state images 71 to 76 correspond to a transfer condition of the cell included in the notification image 60. Further, the transfer source region 61, the transfer destination region 62, and the arrow 64 correspond to a guide image for guiding for a transfer operation of the cell. Specific configurations of those images are not limited, and may be arbitrarily set. Further, the images and the like of the fertilized egg 5 which is taken by the imaging apparatuses 21a and 21b may be displayed in the notification image 60.

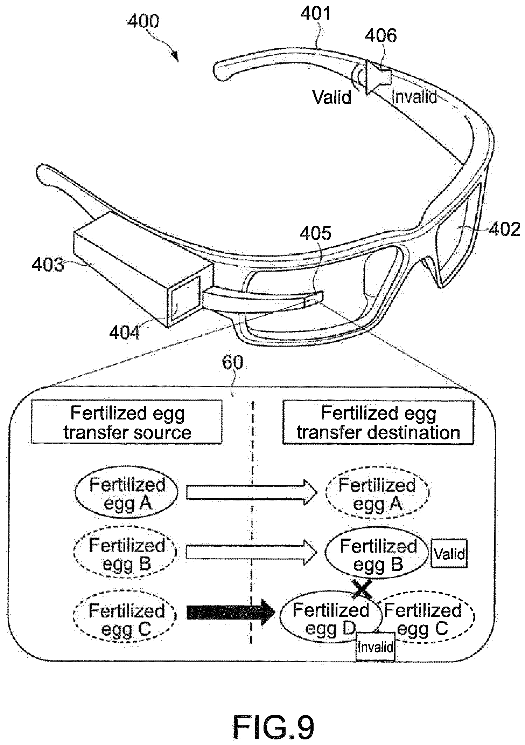

Further, the present technology is not limited to the case where only the transfer condition of the one fertilized egg A is displayed, and respective transfer conditions of a plurality of fertilized eggs on which transfer operations are performed, determination results as to the normality or abnormality of the transfer operations, and the like may be displayed as a list (see Fig. 9).

As described above, in the culture system 100 according to this embodiment, the information processing apparatus 30 compares the first feature quantity extracted from the image of the fertilized egg 5a before transfer and the second feature quantity extracted from the image of the fertilized egg 5b after transfer with each other. With this configuration, it is possible to easily determine whether the fertilized egg 5a before transfer and the fertilized egg 5b after transfer are the same fertilized eggs. As a result, it is possible to sufficiently prevent mix-up and the like of the fertilized eggs 5.

The cells such as the fertilized eggs 5 experience cleavage as the culture progresses, and fragmentation or the like occurs. By extracting and comparing such states of the cells as the feature quantities on a basis of the machine learning algorithm, it is possible to highly accurately determine whether or not those are the same cells. As a result, it is possible to highly accurately determine the normality or abnormality of the transfer operation.

Further, on a basis of the images of the culture vessels 10a and 10b of the transfer source and the transfer destination, the feature quantities of the culture vessels 10 are extracted. With this configuration, it is possible to sufficiently prevent mix-up of the fertilized eggs 5 at the transfer source and mistaking of the culture vessels 10 at the transfer destination. Further, the notification images 60 including the various transfer state images 71 to 76, the guide image, the determination result image 65, and the correction image 69, and the like are displayed and the sounds are output from the speaker 50. That is, positive feed-back and negative feed-back are performed as appropriate in a manner that depends on the normality or abnormality of the transfer operation. As a result, it is possible to sufficiently prevent mix-up and the like, and it is possible to easily correct the operation.

### <Second Embodiment>

A culture system of a second embodiment according to the present technology will be described. In the following description, descriptions of portions similar to the configurations and actions of the culture system 100 described in the above-mentioned embodiment will be omitted or simplified.

### Fig. 6

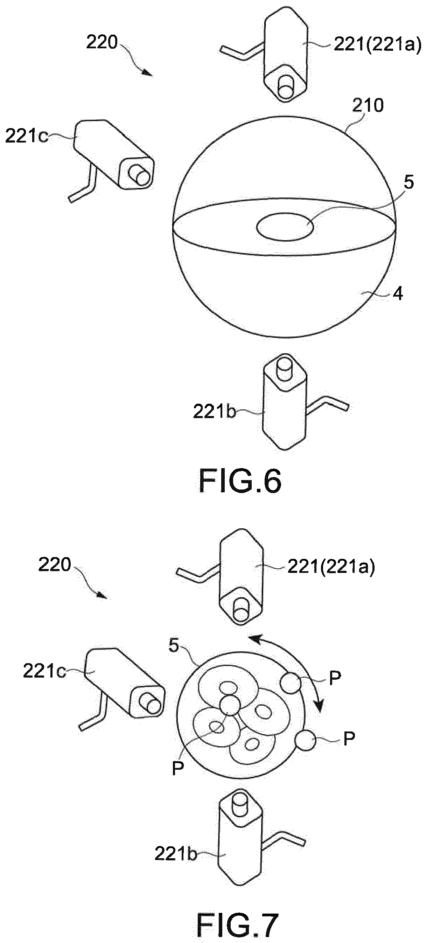

Fig. 6 is a schematic diagram showing a culture vessel 210 and an imaging unit 220 according to this embodiment. Configurations of a culture vessel 210 and an imaging unit 220 to be described below are common to both of a transfer source and a transfer destination.

In this embodiment, the culture vessel 210 having an approximately spherical shape and light transmittance is used. A culture medium 4 and a fertilized egg 5 are put therein. Further, a plurality of imaging apparatus 221 are arranged at a plurality of positions around the culture vessel 210. With this configuration, it is possible to acquire a plurality of images by imaging the fertilized egg 5 in a plurality of directions.

In the example shown in Fig. 6, a first imaging apparatus 221a and a second imaging apparatus 221b are arranged so as to be opposed to each other in a vertical direction. Further, a third imaging apparatus 221c is arranged so as to be capable of imaging the fertilized egg 5 from obliquely above. The three imaging apparatuses 221 and the culture vessel 210 having an approximately spherical shape are configured to be rotatable relative to each other. Thus, in this embodiment, the fertilized egg 5 can be imaged in all directions (360 degrees).

The above-mentioned visible-light cameras can be used as the first to third imaging apparatuses 221a to 221c. Otherwise, infrared ray (IR) cameras or polarization cameras may be used. One kind of camera of the visible-light camera, the IR camera, and the polarization camera may be used. Alternatively, any two kinds of cameras or all kinds of cameras may be used at the same time.

The visible-light camera is high resolution and an image sensor is relatively inexpensive, and thus the cost can be reduced if many visible-light cameras are used. However, the visible-light camera is not suitable for imaging in the dark. The IR camera is capable of visualizing heat, and thus the IR camera is suitable for biological observation. Further, the IR camera is capable of imaging in the dark. The polarization camera is capable of detecting the direction and irregularities of a surface of an observation target. However, with the polarization camera, it is relatively difficult to control a light source and imaging in the dark is difficult.

For example, the imaging apparatuses 221 to be used may be selected as appropriate in view of those characteristics of the respective imaging apparatuses. As a matter of course, the type(s) of the imaging apparatuses 221 may be selected in accordance with the kind of cell that is an observation target, the kind of feature quantity that should be extracted, and the like.

The images of the fertilized egg 5 and the images of the culture vessel 210 can be acquired in all directions with respect to the fertilized egg 5, and thus it is possible to extract useful feature quantities with which the fertilized egg 5 and the culture vessel 210 can be highly accurately identified. As a result, it is possible to highly accurately determine the normality or abnormality of the transfer operation, and it is possible to sufficiently prevent mix-up and the like.

It should be noted that sufficient effects can be obtained not only in the case where the fertilized egg 5 can be imaged in all directions, but also in a case where the fertilized egg 5 can be imaged in a plurality of predetermined directions.

Fig. 7 is a diagram for describing a posture of the fertilized egg 5. The posture of the fertilized egg 5 may be detected on a basis of the image of the fertilized egg 5 which is taken by the imaging unit 220. The posture of the fertilized egg 5 before transfer is detected from the image of the fertilized egg 5 before transfer. The posture of the fertilized egg 5 after transfer is detected from the image of the fertilized egg 5 after transfer.

For example, characteristic points/regions of the fertilized egg 5 are detected as feature points P from the image of the fertilized egg 5. Examples of the feature points P can include a corner of an edge of each blastomere, a coupling portion of each blastomere, and the like. On a basis of the images of the fertilized egg 5 which are taken in all directions, the detected feature points P are tracked in different viewpoints. With this configuration, it is possible to estimate the motion and the posture of the fertilized egg 5. With the estimated posture information and the images taken in all directions, an image of the fertilized egg 5 at an arbitrary time/angle can be acquired.

The feature points P can be detected by, for example, any corner detection, blob detection, and the like. For example, the feature points P can be highly accurately extracted by using a detection method in a manner that depends on the type(s) of the imaging apparatuses 221 to be used. It should be noted that those feature points P are parameters different from the first feature quantity and the second feature quantity of the fertilized egg 5 which have been described above. As a matter of course, the positions of the feature points P, the number of the feature points P, and the like can be reflected on extraction of the first feature quantity and the second feature quantity.

The extraction unit extracts the first feature quantity and the second feature quantity on a basis of the posture information. For example, an image in which a predetermined posture of the fertilized egg 5 is captured is selected as appropriate, and a first feature quantity and a second feature quantity are respectively extracted from that image. With this configuration, the feature quantities of the fertilized egg 5 in the same posture are compared with each other, and thus the matching determination of the fertilized eggs 5 can be highly accurately performed.

Alternatively, the extracted feature quantity and the posture information at that time may be stored in association with each other. The comparison unit reads and compares the first feature quantity and the second feature quantity of the fertilized egg in the same posture. With this configuration, the same effects are provided.

### <Third Embodiment>

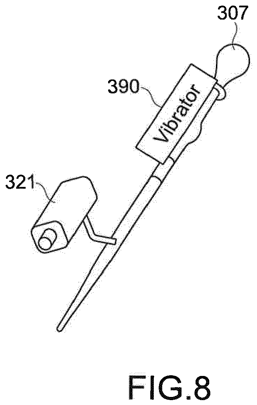

Fig. 8 is a schematic diagram showing a transfer tool to be used in a culture system according to a third embodiment. In this embodiment, a transfer tool 307 is provided with an imaging apparatus 321 and a vibrator 390. The imaging apparatus 321 is positioned such that the imaging apparatus 321 can image an end part of the transfer tool 307. The vibrator 390 is provided near a position at which the embryologist holds the transfer tool 307. A specific configuration of the vibrator 390 is not limited. For example, a vibrator including a vibration motor, a piezoelectric element, and the like is used.

The imaging apparatus 321 makes it possible to take an image of a fertilized egg being transferred. The taken image of the fertilized egg is sent to the information processing apparatus, and the third feature quantity of the fertilized egg being transferred is extracted by the extraction unit. If it is determined that the first feature quantity of the fertilized egg before transfer is unequal to the third feature quantity of the fertilized egg being transferred, a vibration indicating that mix-up occurs is generated by the vibrator 390. That is, the negative feed-back is performed. With this configuration, it is possible to sufficiently prevent mix-up.

In the case where the images of the fertilized egg in all directions can be taken at the transfer source and the transfer destination, i.e., in the case where the posture information can be acquired, it is possible to compare the feature quantities in a manner that depends on the posture of the imaged fertilized egg being transferred irrespective of the direction and the like of the transfer tool 307. With this configuration, it is possible to highly accurately identify the fertilized egg.

It should be noted that the imaging apparatus 321 may take images of the fertilized egg and the culture vessel when the fertilized egg is picked up. It is possible to determine whether or not a correct fertilized egg is picked up by comparing the first and third feature quantities with each other or comparing the feature quantities of the culture vessels with each other.

If the first and third feature quantities are approximately equal to each other and the second and third feature quantities are approximately equal to each other, a vibration indicating that the transfer operation is normally performed is generated by the vibrator. That is, the positive feed-back is performed. With this configuration, it is possible to easily grasp a correct transfer operation.

The present technology is not limited to the case where the imaging apparatus 21a at the transfer source and the imaging apparatus 21b at the transfer destination as shown in Fig. 1 are combined and used with the imaging apparatus 321 of the transfer tool 307, and either the imaging apparatus 21a or 21b may be combined with the imaging apparatus 321 of the transfer tool 307. Otherwise, in a case where only the imaging apparatus 321 of the transfer tool 307 is used, the normality or abnormality of the transfer operation can also be determined. In this case, the imaging apparatus 321 takes either the fertilized egg (culture vessel) before transfer or the fertilized egg (culture vessel) after transfer or both the images of the fertilized egg (culture vessel) before transfer and the fertilized egg (culture vessel) after transfer.

Also regarding the notification method of the determination result, the display of the notification image and the output of the sound may be combined and performed with the notification with the vibration or only the notification with the vibration may be performed. By using at least one of the image, the sound, or the vibration, it is possible to sufficiently makes notification of the determination result.

### <Another Embodiment>

The present technology is not limited to the above-mentioned embodiments, and various other embodiments can be realized.

For example, Fig. 9 is a perspective view showing a configuration example of a head-mounted display (HMD) as an information processing apparatus according to another embodiment. An HMD 400 includes an eyeglass-type frame 401, lenses 402, a processor main body 403, an imaging apparatus 404, a display 405, and a speaker 406.

Control blocks including a CPU, a ROM, and the like are configured inside the processor main body 403, and the extraction unit 31, the comparison unit 32, the determination unit 33, and the notification unit 34 which are shown in Fig. 1 are realized. The notification image 60 including the transfer state image regarding the fertilized egg to be transferred and the determination result image are displayed on the display 405. Further, feed-back with a sound is performed through the speaker 406. By wearing the HMD 400, the embryologist can perform transfer operations and other work while checking the transfer condition and the like of each fertilized egg. As a result, efficient work is realized while sufficiently preventing mix-up and the like.

The imaging apparatus 404 of the HMD 400 may take images of the culture vessels and images of work regions including peripheries, transfer paths and the like of the culture vessels. On a basis of those images, the feature quantities including the identification information of the culture vessels, the positions of the culture vessels and the fertilized eggs, and the like may be extracted. Such information may be used for determination as to the normality or abnormality of the transfer operation. Alternatively, the imaging apparatus 404 of the HMD 400 may take images of the fertilized eggs and extract the above-mentioned first to third extraction quantities.

Hereinabove, the optical image obtained by imaging the cell has been shown as an example of the observation data of the cell. The observation data is not limited thereto. For example, a detection result obtained by a device such as a temperature sensor, a wavefront sensor or the like may be used as the observation data. The first to third feature quantities and the like may be extracted on a basis of that detection result.

Hereinabove, the case where the information processing method according to the present technology is executed by the computer such as the PC operated by the user has been described. However, the information processing method and the program according to the present technology may be executed by another computer capable of communicating with the computer operated by the user via a network or the like. Further, the culture system according to the present technology may be constructed by cooperation of the computer operated by the user and the other computer.

That is, the information processing method and the program according to the present technology may be executed not only in a computer system configured by a single computer but also in a computer system in which a plurality of computers cooperatively operate. It should be noted that in the present disclosure, the system means an aggregate of a plurality of components (apparatus, module (parts), and the like) and it does not matter whether or not all the components are housed in the same casing. Therefore, a plurality of apparatuses housed in separate casings and connected to one another via a network and a single apparatus having a plurality of modules housed in a single casing are both the system.

The execution of the information processing method and the program according to the present technology by the computer system includes, for example, both of a case where the extraction and comparison of the first to third feature quantities and the like, the determination as to the normality or abnormality of the transfer operation, or the output of the notification image and the like are executed by a single computer and a case where those processes are executed by different computers. Further, the execution of the respective processes by a predetermined computer includes causing the other computer to some or all of those processes and acquiring results thereof.

That is, the information processing method and the program according to the present technology are also applicable to a cloud computing configuration in which one function is shared and cooperatively processed by a plurality of apparatuses via a network.

At least two features of the features according to the present technology which have been described above may be combined. That is, the various features described in the respective embodiments may be arbitrarily combined across the respective embodiments. Further, the above-mentioned various effects are merely exemplary and not limitative, and further other effects may be provided.

It should be noted that the present technology can also take the following configurations.
(1) An information processing apparatus, including:
   a first extraction unit that extracts, from observation data of a cell before transfer, a first feature quantity of the cell before transfer;
   a second extraction unit that extracts, from observation data of a cell after transfer, a second feature quantity of the cell after transfer; and
   a comparison unit that compares the extracted first feature quantity and the extracted second feature quantity with each other.
(2) The information processing apparatus according to (1), in which
   the observation data of the cell is an image of the cell, which is taken by one or more imaging apparatuses.
(3) The information processing apparatus according to (2), in which
   the one or more imaging apparatuses include at least one of a visible-light camera, an infrared camera, or a polarization camera.
(4) The information processing apparatus according to any one of (1) to (3), in which
   the observation data of the cell is a plurality of images obtained by imaging the cell in a plurality of directions.
(5) The information processing apparatus according to any one of (1) to (4), in which
   the observation data of the cell is a moving image obtained by imaging the cell in a time series.
(6) The information processing apparatus according to any one of (1) to (5), in which
   the first extraction unit and the second extraction unit respectively extract the first feature quantity and the second feature quantity on a basis of a predetermined machine learning algorithm.
(7) The information processing apparatus according to any one of (1) to (6), in which
   the first extraction unit detects, from the observation data of the cell before transfer, a posture of the cell before transfer and extracts the first feature quantity on a basis of the detected posture, and
   the second extraction unit detects, from the observation data of the cell after transfer, a posture of the cell after transfer and extracts the second feature quantity on a basis of the detected posture.
(8) The information processing apparatus according to any one of (1) to (7), further including
   a determination unit that determines whether or not the transfer of the cell is normal on a basis of a comparison result of the comparison unit.
(9) The information processing apparatus according to any one of (1) to (8), in which
   the first extraction unit extracts a feature quantity of a vessel before transfer, the feature quantity relating to the vessel in which the cell before transfer is put,
   the second extraction unit extracts a feature quantity of a vessel after transfer, the feature quantity relating to the vessel in which the cell after transfer is put, and
   the determination unit determines whether or not the transfer of the cell is normal on a basis of the extracted feature quantity of the vessel before transfer and the extracted feature quantity of the vessel after transfer.
(10) The information processing apparatus according to any one of (1) to (9), further including
   a third extraction unit that extracts, from observation data of a cell being transferred, a third feature quantity of the cell being transferred.
(11) The information processing apparatus according to any one of (1) to (10), further including
   a notification unit that makes notification of a determination result of the determination unit.
(12) The information processing apparatus according to (11), in which
   the notification unit makes notification of the determination result by using at least one of an image, a sound, or a vibration.
(13) The information processing apparatus according to (12), in which
   the notification unit outputs a notification image including the determination result.
(14) The information processing apparatus according to (13), in which
   the notification image includes at least one of a transfer condition of the cell or a guide image for guiding for a transfer operation of the cell.
(15) The information processing apparatus according to any one of (11) to (14), in which
   the notification unit displays a correction image for correcting to normal transfer if it is determined that the transfer of the cell is not normal. Reference Signs List

- 5, 5a, 5b: fertilized egg
- 7, 307: transfer tool
- 10, 10a, 10b, 210: culture vessel
- 20, 220: imaging unit
- 30: information processing apparatus
- 31: extraction unit
- 32: comparison unit
- 33: determination unit
- 34: notification unit
- 35: fertilized egg information DB
- 40: display apparatus
- 50: speaker
- 60: notification image
- 65: determination result image
- 69: correction image
- 71 to 76: first to sixth transfer state images
- 100: culture system
- 321: imaging apparatus
- 390: vibrator
- 400: HMD

## Claims

1. An information processing apparatus, comprising:
a first extraction unit that extracts, from observation data of a cell before transfer, a first feature quantity of the cell before transfer;
a second extraction unit that extracts, from observation data of a cell after transfer, a second feature quantity of the cell after transfer; and
a comparison unit that compares the extracted first feature quantity and the extracted second feature quantity with each other.

2. The information processing apparatus according to claim 1, wherein
the observation data of the cell is an image of the cell, which is taken by one or more imaging apparatuses.

3. The information processing apparatus according to claim 2, wherein
the one or more imaging apparatuses include at least one of a visible-light camera, an infrared camera, or a polarization camera.

4. The information processing apparatus according to claim 1, wherein
the observation data of the cell is a plurality of images obtained by imaging the cell in a plurality of directions.

5. The information processing apparatus according to claim 1, wherein
the observation data of the cell is a moving image obtained by imaging the cell in a time series.

6. The information processing apparatus according to claim 1, wherein
the first extraction unit and the second extraction unit respectively extract the first feature quantity and the second feature quantity on a basis of a predetermined machine learning algorithm.

7. The information processing apparatus according to claim 1, wherein
the first extraction unit detects, from the observation data of the cell before transfer, a posture of the cell before transfer and extracts the first feature quantity on a basis of the detected posture, and
the second extraction unit detects, from the observation data of the cell after transfer, a posture of the cell after transfer and extracts the second feature quantity on a basis of the detected posture.

8. The information processing apparatus according to claim 1, further comprising
a determination unit that determines whether or not the transfer of the cell is normal on a basis of a comparison result of the comparison unit.

9. The information processing apparatus according to claim 1, wherein
the first extraction unit extracts a feature quantity of a vessel before transfer, the feature quantity relating to the vessel in which the cell before transfer is put,
the second extraction unit extracts a feature quantity of a vessel after transfer, the feature quantity relating to the vessel in which the cell after transfer is put, and
the determination unit determines whether or not the transfer of the cell is normal on a basis of the extracted feature quantity of the vessel before transfer and the extracted feature quantity of the vessel after transfer.

10. The information processing apparatus according to claim 1, further comprising
a third extraction unit that extracts, from observation data of a cell being transferred, a third feature quantity of the cell being transferred.

11. The information processing apparatus according to claim 1, further comprising
a notification unit that makes notification of a determination result of the determination unit.

12. The information processing apparatus according to claim 11, wherein
the notification unit makes notification of the determination result by using at least one of an image, a sound, or a vibration.

13. The information processing apparatus according to claim 12, wherein
the notification unit outputs a notification image including the determination result.

14. The information processing apparatus according to claim 13, wherein
the notification image includes at least one of a transfer condition of the cell or a guide image for guiding for a transfer operation of the cell.

15. The information processing apparatus according to claim 11, wherein
the notification unit displays a correction image for correcting to normal transfer if it is determined that the transfer of the cell is not normal.

16. An information processing method, comprising:
by a computer system,
extracting, from observation data of a cell before transfer, a first feature quantity of the cell before transfer;
extracting, from observation data of a cell after transfer, a second feature quantity of the cell after transfer; and
comparing the extracted first feature quantity and the extracted second feature quantity with each other.

17. A program that causes a computer system to execute:
a step of extracting, from observation data of a cell before transfer, a first feature quantity of the cell before transfer;
a step of extracting, from observation data of a cell after transfer, a second feature quantity of the cell after transfer; and
a step of comparing the extracted first feature quantity and the extracted second feature quantity with each other.
